# EUROPEAN PATENT APPLICATION

(11) **EP 2 927 685 A1**
(43) Date of publication of application: **07.10.2015**
(21) Application number: 14163268.7
(22) Date of filing: 02.04.2014
(51) Int. Cl.: G01N 33/50, C12N 5/071

(54) **Suitable hepatocytes for in-vitro hepatitis tests**

(71) Applicant: Medicyte GmbH, 69120 Heidelberg (DE)
(72) Inventor: Küpper, Jan-Heiner, 01968 Kleinkoschen (DE)
(74) Representative: Simandi, Claus

(57) **Abstract**

The invention relates to a method for propagating or enriching of hepatitis infected primary hepatocytes without tumorous characteristics, and to the subsequent use thereof, in particular for in-vitro hepatitis test.

## Description

The invention relates to a method for propagating or enriching of hepatitis infected primary hepatocytes without tumorous characteristics, and to the subsequent use thereof, in particular for in-vitro hepatitis test.

Providing cells that are suitable for in-vitro hepatitis tests presents a medical challenge, whether these cells are intended for use in the development of in-vitro cell systems including corresponding cell cultures. Moreover, there is an urgent need to establish such cell cultures that are as close as possible to human cells, which can then be used for pharmaceutical testing, research, etc.

Particularly, the invention relates to a method for carrying out in-vitro hepatitis tests of chemical, biological and physical active substances or agents with the aid of cell culture systems of proliferating physiologically active liver cells.

The liver is the main and largest inner organ responsible for the modification, clearance, and transformational toxicity of most xenobiotics, serving as a focal point of carbohydrate, lipid, protein and xenobiotic metabolism. In fact, drug induced liver toxicity is the predominant cause of post-market drug withdrawal (Kaplowitz, N. Idiosyncratic drug hepatotoxicity, Nat Rev Drug Discov 4, 489-499 (2005)).

The present state of the art and current gold standard of liver metabolism is culture of primary human hepatocytes. Primary human hepatocytes can be maintained in suspension for a few hours, or stabilized for weeks in oxygenated or microfabricated co-cultures (Ikeda, Y. et al. Long-term survival and functional maintenance of hepatocytes by using a microfabricated cell array, Colloids Surf B Biointerfaces 97, 97-100 (2012)). Primary hepatocyte cultured under these conditions display gene expression and metabolic function equivalent to in vivo. However, while primary hepatocytes are capable of many cycles of replication in vivo, this proliferative capacity is lost in culture (D. Runge et al., Biochem Biophys Res Commun 2000, 274, 1-3). Attempts to expand rodent hepatocytes using co-culture and soluble factors have been moderately successful, but have thus far been unsuccessful in expanding human hepatocyte in vitro. Just recently, a high-throughput screen identified small molecules with the potential of inducing limited proliferation of primary human hepatocytes in culture (Shan, J. et al. Identification of small molecules for human hepatocyte expansion and iPS differentiation, Nat Chem Biol 9, 514-520 (2013))

The scarcity of primary human hepatocytes and the inability to expand them in vitro generated interest in alternative cell models. Hepatoma cell lines, such as HepG2 and Huh7, as well as hTERT-immortalized hepatocytes are readily available. However, immortalized cells rapidly de-differentiate in culture displaying fetal markers such as alpha-fetoprotein (AFP), and only limited mature gene expression and metabolic activity (SL Nyberg et al., Ann Surg 1994, 220, 59-67). Attempts to differentiate induced pluripotent or embryonic stem cells to hepatocytes raise similar problems (Sullivan, G.J. et al. Generation of functional human hepatic endoderm from human induced pluripotent stem cells, Hepatology 51, 329-335 (2010)). Differentiated cells consistently show immature hepatic markers characteristic of fetal hepatocytes and limited mature CYP450 activity. In this context, the recent derivation of the HepaRG cell line from a single tumor of a hepatitis C virus (HCV) patient was a unique event (Le Vee, M. et al. Functional expression of sinusoidal and canalicular hepatic drug transporters in the differentiated human hepatoma HepaRG cell line, Eur J Pharm Sci 28, 109-117 (2006)).

HepaRG cells can be serially passaged, frozen and thawed showing little mature function, but can be differentiated into highly functional cells by long-term treatment with 2% DMSO. It is clear, that a robust method to induce proliferation in multiple genotypes of primary human hepatocytes without loss of function is surly needed.

So called primary hepatocytes, the parenchymal cells of the liver, are the main cell type responsible for drug metabolism due to their abundance in phase I/II metabolic enzymes. Regretfully, primary human hepatocytes do not proliferate in vitro, while their transformation causes loss of metabolic function. However, proliferating human hepatocytes can undergo 20 to 30 population doublings, and differentiate when contact-inhibited into metabolically functional, polarized cells with active bile canaliculi. The obtained cells lack fetal and tumor markers such as AFP (supra) and show high levels of CYP3A4 expression and function. The obtained cells can be frozen and thawed, and differentiate when contact-inhibited into metabolically functional, polarized cells with active bile canaliculi and basal surface function.

Moreover, differentiated cells show gene expression, CYP450 activity, and TC50 toxicity profile comparable to primary human hepatocytes. Importantly, the present invention discloses for the first time that the proliferating hepatocytes can be readily infected with the Hepatitis C Virus (HCV) showing replication and infectivity profile comparable to the current Huh7.5 culture model (cell line).

The method is particularly suited for the hepatitis testing of both known and new drugs and active substances as well as combinations thereof in humans and animals.

Firstly, drugs, chemicals or biological active substances can, for example, also develop undesirable side effects such as liver damage, damage to the mycoardium, neurotoxicity or teratogenicity, in addition to the desired effect within the meaning of the therapy. In the process, many cells of an organ may be lost, including degenerative organ disease, for example cardiac failure or liver damage. The cause of this toxicity can basically be due to all compartments and functions of a cell becoming damaged or being influenced, which is to say, for example, damage of the cell membrane, influence on physiological processes such as cell respiration, intracellular transport, signal transduction and gene expression, just to name a few examples.

In a very preferred aspect, there is need to identify responder or non-responder for appropriate or potential medicaments for patients suffering from hepatitis. For example, there are well known HCV-patients which do not respond to conventional immunomodulatory / antiviral combination therapy such as ribavirin/interferon therapy ("non-responders") and/or in patients that partially respond to conventional immunomodulatory / antiviral combination therapy such as ribavirin / interferon therapy ("partial responders") and/or in patients that show a robust initial response followed by rebounds of viral titers during or after therapy ("relapsers").

In the connection with conventional hepatitis C therapy by administration of ribavirin / interferon, the terms "nonresponders", "partial responders" and "relapsers" are known to the person skilled in the art. Nowadays, pegylated interferon plus ribavirin therapy for hepatitis C virus fails in approximately half of genotype 1 patients. Treatment failure occurs either by nonresponse (minimal declines in viral titer) or relapse (robust initial responses followed by rebounds of viral titers during or after therapy).

Providing suitable hepatocytes for testing is a medical and diagnostic challenge, in particular in the development of in *vitro* cell systems, including the related cell cultures.

As a result, a great need exists for establishing cell cultures/cell systems that are similar to human cells to the greatest extent possible, so that valid *in vitro* hepatitis test procedures can be carried out, not limited to, for example, the identification of non-responders, partial responders and relapsers. Moreover, there is need to stratify the patients.

Cell lines have become established in the prior art, which are cells that can reproduce without limitation on an appropriate culture medium and are immortal. In particular tumor cells or tumor-like cells are known, such as HeLa cells - the cervical cancer cell line, COS cells, HEK 293 cells - kidney, Chinese hamster ovary (CHO) cells, HEp-2 - the human epithelial larynx carcinoma cell line and many more. The production of such cell lines is described in EP833934 (Crucell), for example Cell lines such as these are used, for example, for drug testing. However, the drawbacks of such cell lines are the genetic changes (such as point mutations, translocations of chromosome parts (rearrangements), an increase in the copy number of genes (gene amplification), and even changes in the sets of chromosomes (aneuploidy)) as well as the tumor properties due to lacking contact inhibition, whereby the cells are empowered into *in vitro* growth on soft agar substrates. Tumor cells additionally can grow an unlimited number of cell divisions that is due to immortalization. It is known that the cells of such cell lines gradually transform over the course of cultivation due to spontaneous mutations and can develop into a malignant cell population and are genetically unstable. Based on the inventors' findings, a critical threshold of accumulated mutations occurs in the culture after only approximately 60 cell divisions. These can be mutations, which lead to the activation of oncogenes or inactivation of tumor suppressor genes.

As a result, cells that can prevail in a cell population are those that exhibit increased cell division activity due to the accumulated mutations. This selection process corresponds to the precancerous condition in the development of tumors; additionally, cell lines that are commercially available usually have already undergone an unknown number of doubling processes, if they do not originate from malignant tumor cells to begin with.

Differentiated hepatocytes, as they are present in an intact liver, in vivo have a variety of functions that are important for this biotransformation of substances in food, but also drugs or toxins (overview in Elaut, G., Henkens, T., Papeleu, P., Snykers, S., Vinken, M., Vanhaecke, T., and Rogiers, V., Molecular mechanisms underlying the dedifferentiation process of isolated hepatocytes and their cultures, Curr Drug Metab 7, 629-660 (2006)). Phase I enzymes of the cytochrome P450 system are important for biotransformation. In humans, numerous isozymes are found, such as CYP1A1, CYP1A2, CYP2A6, CYP2B6, CYP2C8, CYP2C9, CYP2C19, CYP2D6, CYP2E1, CYP3A4, CYP3A5, CYP3A7, CYP4A11, which perform different functions. For some isozymes polymorphisms are known, which can be responsible for the individual variability in the toxic effect of drugs on the liver. CYP450 enzymes are oxidoreductases, which bring about oxidative degradation or metabolization of numerous substances, including pharmaceuticals.

In addition to the Phase I enzymes, Phase II enzymes exist, for example N-acetyltransferases [NATs] as well as UDP-glucoronyltransferases and sulfotransferases.

For assessing the potential liver toxicity of active substance candidates, and of chemicals in general, the functionalities of the CYP450 systems, Phase II enzymes as well as other liver functions are of decisive importance.

Thus, the object of the present invention is to provide suitable hepatocytes for carrying out *in vitro* hepatitis tests.

The object is achieved entirely by claim 1 and other independent claims.

The term "hepatitis infected hepatocytes" refers to the disease hepatitis - known as liver inflammation - caused by an infected agent like virus, fungus, parasite or bacteria. Preferred are viruses, which are known as e.g. hepatitis A Virus (HAV), hepatitis B Virus (HBV), hepatitis C Virus (HCV), hepatitis D Virus (HDV). Viral hepatitis refers to infections that affect the liver and are caused by viruses. It is a major public health issue worldwide. Not only does viral hepatitis carry a high morbidity, but it also stresses medical resources and can have severe economic consequences. The majority of all viral hepatitis cases are preventable.

Viral hepatitis includes six distinct disease entities (hepatitis A, B, C, D, E and G), which are caused by at least five different viruses. Hepatitis A and hepatitis B (infectious and serum hepatitis, respectively) are separate diseases and both can be diagnosed by a specific serologic test. Hepatitis C and E comprise a third category, each a distinct type, with Hepatitis C parenterally transmitted, and hepatitis E enterally transmitted. Hepatitis D, or delta hepatitis, is another distinct virus that is dependent upon hepatitis B infection. This form of hepatitis may occur as a super-infection in a hepatitis B carrier or as a co-infection in an individual with acute hepatitis B.

Hepatitis C is an infectious disease in humans, which is caused by the hepatitis C virus (HCV). HCV infection can lead in its course to severe liver damage, e.g. inflammation of the liver parenchyma, fibrosis of the liver, cirrhosis of the liver and carcinoma of the liver. In over 80% of the infected patients, HCV infection becomes chronic. The transmission of HCV usually takes place parenterally via the blood.

It is estimated that about 170 million people worldwide are infected with the hepatitis C virus (HCV). The infected patients can be asymptomatic for decades, until the development of cirrhosis of the liver and/or hepatocellular carcinomas finally occurs. Approximately 40-50% of the liver transplants in the United States are based on HCV infections. Six genotypes of HCV have been identified (HCV1-HCV6), which differ in their geographical spread and in their response to medicinal therapies. HCV proteins have been shown to induce activation of STAT-3 via oxidative stress and Ca2+ signalling (K Koike et al., Hepatol Res 2006; 34: 65-73; G Waris et al., J Virol 2005, 79, 1569-80) as well as lipid peroxidation products and antioxidant gene expression (M Okuda et al., Gastroenterology 2002, 122, 366-375). It appears that the balance of the oxidative and reductive potentials within the cell (cellular redox state) has profound consequences on signal transduction pathways (YM Janssen et al., Am J Physiol 1997, 273:789-96) including impaired IFN-alpha signalling (D Di Bona et al., J Hepatol. 2006, 45, 271-9).

HCV infection is divided according to ICD10 (WHO, Version 2007) into acute (B17.1) and chronic hepatitis C (B18.2).

HCV is one of the most important causes of the developments of acute or chronic hepatitis. The clinical course of the disease, however, might be very different and subject to a high variability. It is thus not possible to speak about a typical course of the disease, since the HCV infection is essentially manifested by a broad clinical spectrum, i.e. by variable symptoms, different clinical pictures and variable hepatic and extrahepatic secondary diseases.

In approximately 20% of the patients with acute hepatitis, the inflammation of the liver is to be attributed to an HCV infection. In the acute phase, however, hepatitis C usually proceeds asymptomatically and it is therefore not diagnosed in approximately 85% of the cases. In some cases, only non-specific symptoms of a putatively flu-like syndrome occur. Usually, the infection is not manifested during the acute phase.

Hepatitis C becomes chronic in about 85% of the patients with acute HCV infection. This high chronification rate appears to be a result of the high virus variability of the HCV; i.e. the gene which codes for the coat of the HCV is subject to a high mutation rate. Because of the high virus variability, and in particular the high variability of the antigenic epitope of the HCV, the mutated HCV escapes recognition by the human immune system. In about 25% of the patients, as a result of chronic liver inflammation the formation of cirrhosis of the liver occurs with an increased risk of the development of carcinoma of the liver (cf., for example, J. H. Hoofnagle, Hepatology 1997, 26, Suppl. 1, 15S-20S; M. I. Memon et al., Journal of Viral Hepatitis 2002, 9, 84-100; S. L. Tan et al., Nature Reviews, Drug Discovery 2002, 1, 867-81).

Proliferating hepatitis infected hepatocytes surprisingly exhibit the following advantages:
The physiologically relevant properties of the proliferating hepatocytes according to the invention are specified in that these have at least four out of at least six different Phase I enzyme functions, even during the proliferative phase, preferably selected from the group consisting of CYP1A2, CYP2C9, CYP2C19, CYP2D6, CYP2E1 and CYP3A4, which are responsible for approximately 90% of all oxidative metabolization of drugs (Arimoto, R., Computational models for predicting interactions with cytochrome p450 enzyme, Curr. Top. Med. Chem. 6, 1609-1618 (2006)), and therefore in particular also contain more than 6 different Phase I enzymes, in particular ten different Phase I enzymes, preferably CYP1A1, CYP1A2, CYP2A6, CYP2B6, CYP2C8, CYP2C9, CYP2C19, CYP2D6, CYP2E1, CYP3A4, in particular thirteen different Phase I enzymes, in particular CYP1A1, CYP1A2, CYP2A6, CYP2B6, CYP2C8, CYP2C9, CYP2C19, CYP2D6, CYP2E1, CYP3A4, CYP3A5, CYP3A7, CYP4A11.
Additionally, the problem of false positive and false results from the prior art is also completely solved, because these proliferating hepatocytes:
   a. have active Phase I and II activities during proliferation;
   b. have significant activities that correspond to in vivo conditions in the enzyme provision;
   c. have Phase I activities that are maintained over several days;
   d. have enzyme activity that can be induced by way of reagents;
   e. external metabolization by way of microsomes is consequently eliminated;
   f. false positive results from reactive reagents that cannot penetrate cells are considerably reduced; and
   g. after the agent has entered the cell, the agent itself and the resulting metabolite can act on the DNA, and consequently false negative results due to inadequate metabolization of the test substance are eliminated or considerably reduced,
   h. most important, the virus, parasite, bacteria or fungus causing hepatitis are enriched from passage to passage resulting in a sufficient amount for hepatitis testing procedures.

The term "hepatitis infected hepatocytes" relates also to the infection of the hepatocytes, for example, by using the serum of a patient or individual or by isolating already infected hepatocytes from a patient or individual, or in general a donor.

The enrichment of such suitable hepatitis infected hepatocytes is, for example, described in general in the applicant's WO2009030217, which preferably can be obtained from primary cells. Moreover, proliferating hepatocytes can likewise be obtained from other precursor cells, such as stem cells, adult cells and other cells that can be differentiated.

Within the scope of the invention, the term "primary cells" shall be understood to mean explants that are obtained directly from bodily fluids or from bodily tissues of multicellular organisms, such as humans, mammals or suitable donors, and that have normal, which is to say not degenerated, cells. Primary cell cultures are primary cells that have been cultured up to the first passage. Primary cells have natural differentiation properties and are mortal.

So as to maintain cells *in vitro* in an unlimited manner, a method must be employed that compensates for the shortening of chromosomal telomeres that occurs with each cell division. One such option is the use of telomerase (Harley, C. B. and B. Villeponteau. 1995. Telomeres and telomerase in aging and cancer, Curr. Opin. Genet. Dev. 5:249- 255). Cells that are able to compensate for the loss of telomeres, for example by way of telomerase, can grow an unlimited number of cell divisions and have immortality. However, over the course of the cell divisions, there is the inevitable drawback that mutations occur, which sooner or later must lead to the development of cancer.

So as to maintain human primary cells, or cells that can be differentiated, *in vitro,* the following steps can be carried out:
Primary cells or cells that can be differentiated are
   a.) isolated;
   b1.) functionally introduced into the cell with at least one proliferation gene or the gene product thereof;
      and/or
   b2.) inactivated with at least one cellular factor that induces cell division arrest; and/or
   b3.) transiently immortalized;
   c.) cultured and/or passaged.

However, the starting material used is preferably human primary liver cells, which can be obtained by way of biopsy, for example.

Surprisingly, it is now found that hepatitis infected hepatocytes can be also processed in the same manner. Hence, the invention is directed to obtain or enrich hepatitis infected hepatocytes in vitro, the following steps can be carried out:
Hepatitis infected hepatocytes that can be differentiated are
   a.) isolated;
   b1.) functionally introduced into the cell with at least one proliferation gene or the gene product thereof;
      and/or
   b2.) inactivated with at least one cellular factor that induces cell division arrest; and/or
   b3.) transiently immortalized;
   c.) cultured and/or passaged.

Preferably, more than ten additional passages can be carried out as compared to primary cells, or more than 20 to 60 additional passages.

According to the invention, proliferating hepatocytes as described above are obtained, which are highly suited for carrying out hepatitis tests.

Particularly advantageously, hepatocytes can be obtained which do not take on any properties of tumor cells, and more particularly of malignant tumor cells, such as growth in soft agar or tumor growth in vivo (the growth of tumors in xenograft animal models).

Such cells are cultured on culture media that are known to a person skilled in the art.

Within the scope of the present invention, a proliferation gene is one that improves cell division and enables cell division capacity in the primary cell that can be increased to a limited extent, wherein the likelihood of cell transformation or changes of the differentiation properties is drastically reduced as compared to the cell lines from the prior art.

According to the invention, the proliferation gene is preferably selected from the group of viral proliferation genes: large and small tumor antigens (TAgs) of polyomaviruses such as SV40, JK virus and BC virus; E6 and E7 of papillomaviruses such as HPV (human papillomavirus) and BPV (bovine papillomavirus);; the E1A and E1B proteins of adenoviruses, EBNA proteins of the Epstein Barr virus (EBV); as well as the proliferation gene of HTLV and Herpesvirus saimiri and the respective coding proteins or the chimera thereof, selected from the group of the cellular proliferation genes, in particular from the following classes of genes: myc, jun, ras, src, fyg, myb, E2F and Mdm2 and TERT (a catalytic subunit of the enzyme telomerase), preferably human telomerase (hTERT).

However, according to the invention viral proliferation genes are preferred, with E6 and E7 of HPV or BPV or SV40 large and small T-antigen being particularly preferred. To this end, proliferation genes of the HPV type can be used, which are related to malignant diseases. The best known examples of high-risk papillomaviruses are HPV16 and HPV18. Additional examples of the high-risk group include HPV 31, 33, 35, 39, 45, 51, 52, 56, 58, 59, 68, 73 and 82. However, it is also possible to use the E6 and E7 proliferation genes of so-called low-risk HPVs. Known examples include the HPV types 6 and 11, other HPV types of the low-risk group include HPV 40, 42, 43, 44, 54, 61, 70, 72 and 81. Moreover the corresponding chimera or chimeric gene products can be arbitrarily combined and used.

The significance of the SV40 large T-antigen is based on the inactivation of both the p53 and pRB pathway controlling cell cycle checkpoints, DNA repair and apoptosis.

The significance of the E6 proteins in connection with an increase in proliferation lies above all in the inactivation of the p53 pathway and in the induction of telomerase. The significance of the E7 proteins in connection with an increase in proliferation lies above all in the inactivation of the pRb pathway. In connection with the invention, it is also possible to combine the proliferation genes of different serotypes of one virus species or of different virus species or even to produce and use chimeric proliferation genes of different serotypes of one virus species or different virus species. For example, one E6 domain in a chimeric gene can stem from HPV 16, for example, while another stems from HPV 6. Of course the proliferation genes can also be truncated or have one or more base exchanges, without departing from the scope of the invention. The aforementioned proliferation genes represent preferred embodiments and are not intended to limit the invention. The proliferation gene can optionally be the subject matter of a synthetic or artificially produced gene sequence.

These factors are "functionally introduced" into the target cells, the cell division capacity of which is supposed to be increased, and for this purpose the following gene transfer systems may be used, without being limited thereto: transfer of expression constructs of the above-mentioned gene functions into cells by way of the traditional calcium-phosphate method

(Wigler, M. et al., 1977, Cell 11:223-232), by way of lipofection (Felgner, P. L. et al, 1987, Proc. Natl. Acad. Sci. U.S.A 84:7413-7417), by way of electroporation; Wolf, H. et al., 1994, Biophys.J. 66:524-531), by way of microinjection (Diacumakos, E. G. 1973, Methods Cell Biol. 7:287-311), by way of conjugates which are received via cellular receptors or receptor-independently. The above-mentioned gene functions can also be transferred to the target cells by way of viral vectors. Examples include retroviral vectors, AAV vectors, adenovirus vectors and HSV vectors, just to mention a few examples of vectors (overview of viral vectors in: Lundstrom, K. 2004. Technol. Cancer Res.Treat. 3:467-477; Robbins, P. D. and S. C. Ghivizzani, 1998, Pharmacol. Ther. 80:35-47). The term "functionally introduced" comprises in particular the transfection of the target cells by way of at least one proliferation gene.

The expression of the above-mentioned viral or cellular proliferation genes can be controlled by strong or weak constitutive promoters, tissue-specific promoters, or inducible promoters (Meyer-Ficca, M. L. et al. 2004. Anal. Biochem. 334:9-19), or the expression cassettes can be flanked by specific sequences for molecular excision systems. Examples include the Cre/Lox system (US patent 4,959,317), the use of which leads to the molecular removal of the expression constructs from the genome of the target cells.

In a further embodiment, the gene products of the proliferation genes can likewise be functionally introduced directly into the target cell as such or by way of a fusion protein. Preferably these are messenger proteins (transport proteins) such as VP22, HIV TAT (Suzuki et al., 277 J. Biol. Chem. 2437-2443 2002 and Futaki 245 Int. J. polypeptide (WO97/12912 and WO99/11809) or Penetratin (Derossi et al., 8 Trends Cell Biol., 84-87 (1998), Engrailed (Gherbassi, D. & Simon, H. H. J. Neural Transm. Suppl 47-55 (2006), Morgan, R. 580 FEBS Lett., 2531-2533 (2006), Han, K. et al. 10 Mol. Cells 728-732 (2000)) or Hoxa-5 (Chatelin et al. 55 Mech. Dev. 111-117 (1996)), a polymer made of L-arginine or D-arginine amino acid residues (Can. Patent No. 2,094,658; U.S. Pat. No. 4,701,521; WO98/52614), a polymer made of L-lysine or D-lysine amino acid residues (Mai et al., 277 J. Biol. Chem. 30208-30218 (2002), Park et al. 13 Mol. Cells 202-208 (2002), Mi et al. 2 Mol. Ther. 339-347 (2000)), transcription factors such as BETA2 /neuro D, PDX-1 (Noguchi and Matsumoto 60 Acta Med. Okayama 1-11, (2006), Noguchi et al. 52 Diabetes 1732-1737 (2003), Noguchi et al. 332 Biochem. Biophys. Res. Commun. 68-74 (2005)), nuclear localization signal, (Yoneda et al. 201 Exp. Cell Res. 313-320 (1992), histone-derived peptides (Lundberg and Johansson 291 Biochem. Biophys. Res. Comm. 367-371 (2002)), a polymer made of cationic macromolecules, FGF-1 and FGF-2, lactoferrin and the like, as described appropriately in the literature.

The invention therefore also relates to such proliferating hepatocytes which are transiently immortalized, preferably by way of i.) a polypeptide having cell immortalization activity; ii.) a polypeptide that synthesizes telomeric DNA at chromosomal ends, or a respective fusion peptide thereof, wherein the fusion peptide in a first part consists of a transport protein, see above.

Such a polypeptide having cell immortalization activity can, for example, be obtained from the aforementioned viral or cellular proliferation genes. Moreover, reference is made to EP 1174436 B1 for the production of such polypeptides.

Such a polypeptide that synthesizes telomeric DNA at chromosomal ends is preferably selected from the group consisting of telomerase, telomerase reverse transcriptase (hTERT), p140, p105, p48 and p43. Moreover, reference is made to EP 1174436 B1 for the production of such polypeptides.

Within the scope of the present invention, "inactivated with at least one cellular factor that induces cell division arrest" shall be understood to mean that, for example, cell division arrest is activated as part of the senescence program (overview in: Ben Porath, I. and R. A. Weinberg. 2005. Int. J. Biochem. Cell Biol. 37:961-976.) or it refers to the cell division arrest that is activated in cells within the scope of the differentiation program. For example, it is known in the case of cardiac muscle cells that these stop dividing shortly after birth, which is regulated, among other things, by the expression of cell cycle inhibitors such as p16, p21, p27 (Brooks, G., et al. 1998. Cardiovasc. Res. 39, 301-311; Flink, I.L. et al., 1998. J. Mol. Cell Cardiol. 30, 563-578; Walsh, K. and Perlman, H. 1997. Curr. Opin. Genet. Dev. 7, 597-602). Similar processes surely apply to the majority of all primary cell types. Inactivation of cell cycle inhibitors in differentiated cells thus could cause the cells to return to proliferation. In the context of the invention, this also applies to further cell cycle inhibitory proteins not mentioned here.

Within the scope of the invention, the protein p53, which is important for controlling the cell cycle, and all proteins binding directly to p53, upstream and/or downstream factors of this p53 pathway can generally be inactivated so as to achieve the goal of increased cell division capacity (overview of the p53 pathway in: Giono, L. E. and J. J. Manfredi. 2006. J. Cell Physiol 209:13-20; Farid, N. R. 2004. Cancer Treat.Res. 122:149-164).

Within the scope of the invention, the protein P16/INK4a, which is important for controlling the cell cycle, and all proteins binding directly to P16/INK4a, upstream and/or downstream factors of this p16 pathway can generally be inactivated so as to achieve the goal of increased cell division capacity (overview of the p16/INK4a pathway in: Shapiro, G. I. et al., 2000. Cell Biochem. Biophys. 33:189-197).

Within the scope of the invention, the protein pRb, which is important for controlling the cell cycle, and all members of the pRb family (for example p107, p130) and all proteins binding directly to members of the pRb family, upstream and/or downstream factors of this pRb pathway can generally be inactivated so as to achieve the goal of increased cell division capacity (overview of the pRb pathway in: Godefroy, N. et al. 2006. Apoptosis. 11:659-661; Seville, L. L. et al. 2005. Curr. Cancer Drug Targets. 5:159-170).

Inactivation of cellular factors such as p53, pRb, p16 and the like can, for example, take place by the expression of dominant negative mutants of the corresponding factors (Herskowitz, I. 1987. Nature 329:219-222; Küpper, J. H., et al. 1995. Biochimie 77:450-455), by the inhibition of gene expression of these factors with the aid of antisense oligonucleotides (Zon, G. 1990. Ann. N.Y. Acad. Sci. 616:161-172), RNAi molecules (Aagaard, L. and J. J. Rossi. 2007. Adv. Drug Deliv. Rev. 59:75-86; Chakraborty, C. 2007. Curr. Drug Targets. 8:469-482), morpholinos (Angerer, L. M. and R. C. Angerer. 2004. Methods Cell Biol. 74:699-711), ribozymes (Sioud, M. and P.

O. Iversen. 2005. Curr. Drug Targets. 6:647-653) or by way of gene knockout (Le, Y. and B. Sauer. 2000. Methods Mol.

Biol. 136:477-485; Yamamura, K. 1999. Prog. Exp. Tumor Res. 35:13-24). These methods are known to a person skilled in the art and described in many places in literature. Inactivation can also take place by the action of specific antibodies (for example single chain antibodies, intrabodies and the like; overview in: Leath, C. A., III, et al. 2004. Int. J. Oncol. 24:765-771; Stocks, M. R. 2004. Drug Discov. Today 9:960-966) . Inactivation can also take place by the use of chemical inhibitors of the cellular factors, for example by the use of kinase inhibitors. One example of a kinase inhibitor is the substance imatinib (Gleevec^{®}). A reduction in cell proliferation is achieved this way. Imatinib is a specific inhibitor that blocks the activity of Abl tyrosine kinase in diseased cells and thereby suppresses a pathologically increased proliferation of mutated blood stem cells.

In a preferred embodiment, the invention thus likewise relates to a method for producing an assay or test system, comprising the following steps:
a.) providing a substrate material;
b.) immobilizing or fixing or suspending proliferating hepatocytes on this substrate material;
c.) infecting the hepatocytes from b.) with bodily fluid from a patient suffering from hepatitis and obtaining hepatitis infected hepatocytes and culturing or enriching;
c.') optionally portioning obtained hepatitis infected hepatocytes resulting in one or more variant of the hepatitis infected hepatocytes;
   bringing at least one cell from c.) or c'.) in contact with an agent.

In a preferred embodiment, the invention thus likewise relates to a method for producing a cell bank or cell population comprising proliferating hepatitis infected hepatocytes according to step c.) or c.') as above captioned. The advantage of the said method is that several variants of proliferating hepatitis infected hepatocytes can be obtained. Hence, the present invention is directed to a method for producing a cell bank, comprising the following steps:
a.) providing a substrate material;
b.) immobilizing or fixing or suspending proliferating hepatocytes on this substrate material;
c.) infecting the hepatocytes from b.) with bodily fluid from a patient suffering from hepatitis and obtaining hepatitis infected hepatocytes and culturing or enriching;
c.') optionally portioning obtained hepatitis infected hepatocytes resulting in one or more variant of the hepatitis infected hepatocytes.

In a further preferred embodiment of the present invention relates to a method for producing a cell bank and a method for characterization of an external cell, comprising the following steps:
a.) providing a substrate material;
b.) immobilizing or fixing or suspending proliferating hepatitis infected hepatocytes on this substrate material,
c.) obtaining several variants of proliferating hepatitis infected hepatocytes through culturing and optionally portioning,
d.) optionally, bringing at least one cell from c.) in contact with an agent or
e.) optionally, comparing at least one cell from c.) with an external hepatocyte in order to identify the variant of the hepatitis infected hepatocytes.

In a very preferred aspect of the invention, the said external hepatocyte (e.g. derived from biopsy) may be derived from a patient suffering from a liver inflammation, particularly hepatitis.

Methods for comparison of two and more cells or infected hepatocytes are well known in the art and may embrace PCR, DNA / RNA sequencing, proteomics, mass-spectrometry, protein-analysis including enzyme assays and signal transduction analyses. In a preferred aspect the DNA/RNA of the infected cells shall be compared each other in order to determine the variant of the hepatitis infected hepatocytes.

The above defined method allows advantageously the identification of an appropriate agent in order to cure a liver inflammation, particularly hepatitis, of the related patient or individual.

The term "variant of the hepatitis infected hepatocytes" relates to the fact that particularly viruses may alter from one generation of hepatitis infected hepatocytes to another generation or may alter within one generation, which may result in different variants.

Within the scope of the present invention, an agent refers to any arbitrary substance, for example drugs and drug candidates that are approved or in development, and the precursors thereof; chemicals in general; biological active substances, which is to say molecules generated by cells, such as proteins that occur naturally this way, or in modified form in organisms or viruses, or can be formed there; including under physical effects such as electromagnetic radiation, heat, cold energy, sound or the like. The term "bodily fluid" shall mean blood, serum or any other fluid from a patient suffering from hepatitis. The said fluid is proper to infect a hepatocyte.

A change in the status of a hepatitis infected hepatocyte takes place as a result of the effect(s) of one or more agents. On the other hand, a change in the status may manifest itself in changed activity, which results in a changed metabolism of the hepatitis infected hepatocyte.

Hence the said agent may result in the identification, characterization or verification of an potential or existing medicament, in particular a virustatic or antiviral, more preferably viral load reducing medicament for the treatment of viral hepatitis, in particular of hepatitis B or C, in particular of chronic or acute hepatitis C virus infections.

Any event can be determined in a broader sense with an assay reagent, for example by way of a fluorescence-labeled antibody or the like. In particular suitable bioanalytical methods should be mentioned here, for example immunohistochemistry, antibody arrays, Luminex / Luminol, ELISA, immunofluorescence, and radioimmunoassays. A preferred event is of course an increasing or decreasing titer of the virus, fungus, parasite, bacteria causing hepatitis.

Hence, the said assay or cell bank can be used to identify patients or individuals, which may suffer from hepatitis caused by virus, fungus, parasite, bacteria. Moreover, the inventive hepatitis testing procedures allows stratifying of patients, in particular in responder, non-responder or relapser. Therefore, the invention refers to the use of an assay comprising proliferating hepatitis infected hepatocytes for identifying, stratifying of patients or individuals, in particular in responder, non-responder or relapser.

According to the invention, the term "stratification" comprises the identification of patients, particularly risk patients, having poor prognoses, for the purpose of more intensive diagnostics and therapy/treatment of hepatitis with the goal of enabling the most favorable course of the disease possible. Risk stratification according to the invention consequently allows an effective treatment method or therapy, which exists for example in the case of hepatitis in the form of newer drugs.

Hence, the invention relates to the use of an assay or cell bank or method for risk stratification of hepatitis for the execution of clinical decisions, particularly advanced treatments and therapies using drugs, particularly in intensive care or emergency care, to include the decision to hospitalize the patient.

Hence, the invention relates to the use of an assay or cell bank or method for the risk stratification of hepatitis for the prognosis, early detection and detection by differential diagnosis, assessment of the severity, and assessment of the course of the disease concomitant with the therapy.

The term "substrate material" comprises embodiments such as a filter, a membrane, a magnetic spherule, a silicon wafer, glass, plastic material, metal, a chip, a mass spectrometry target, or a matrix, for example made of proteins, or other matrices, such as PEG for example, and the like.

In a further preferred embodiment of the arrangement according to the invention (synonym: array), this array corresponds to a lattice having the size of a microtiter plate (96 wells, 384 wells or more), a silicon wafer, a chip, a mass spectrometry target or a matrix.

The substrate material (matrix) can be present in the form of spherical, non-aggregated particles, referred to as beads, fibers or a membrane, wherein the porosity of the matrix increases the surface.

Detailed description of the invention:

Induction of reversible proliferation in primary human hepatocytes

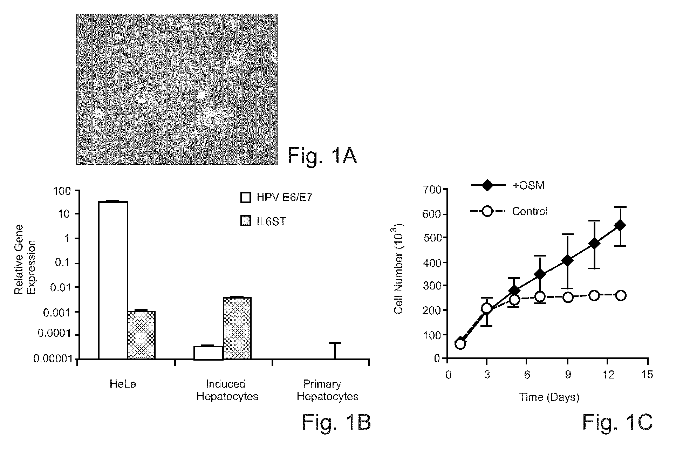

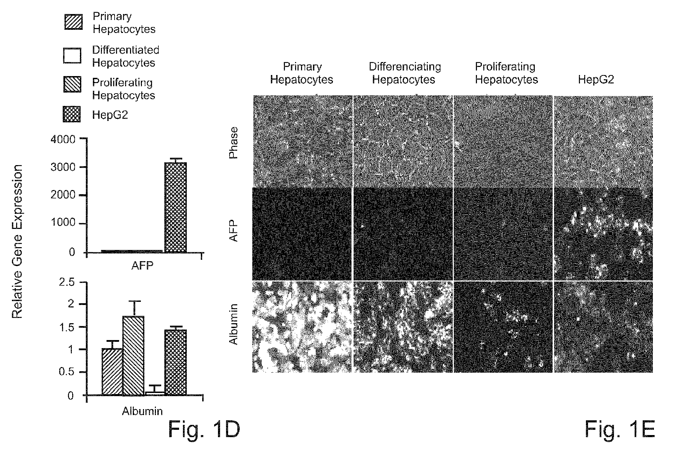

Primary human hepatocytes fail to proliferate in vitro, and their immortalization causes de-differentiation and loss of function partially due to epithelial-mesenchymal transition (EMT) (D Runge et al., Biochem Biophys Res Commun 2000, 274, 1-3). To circumvent this primary human hepatocyte were transduced with HPV E6 and E7 genes shown to induce growth regulation in a p53-independent manner (Lopez-Ocejo, O. et al. Oncogenes and tumor angiogenesis: the HPV-16 E6 oncoprotein activates the vascular endothelial growth factor (VEGF) gene promoter in a p53 independent manner. Oncogene 19, 4611-4620 (2000)). Ten days after transduction, proliferating colonies were evident (Fig. 1A). To stabilize the epithelial phenotype of the cells, cultures were treated with MEK1/2 inhibitor U0126 for one to three weeks, resulting in a stable E-Cadherin positive epithelial phenotype, and negative expression of N-Cadherin and SNAIL characteristic of EMT in transformed cells such as HepG2. Cells not treated with U0126 adopt a fibroblastoid phenotype. Gene expression analysis shows that induced hepatocyte express 1x10^6 -fold less HPV E6/E7 than HeLa cells, a line that was transformed by HPV infection. Interestingly, the cells show marked up-regulation of IL6ST, the OSM receptor (Fig. 1B). Stimulation with OSM causes marked proliferation of sub-confluent cells, with doubling time of 39 hours, until the culture reaches confluence (Fig. 1C). Upon reaching confluence, OSM is removed, and the cells cease to proliferate (Fig. 1C). The cultures differentiate, acquiring a cuboidal morphology with bright cell borders reminiscent of primary human hepatocytes (Fig. 1E). Transformed human hepatocytes could be frozen, thawed, and serially passaged by standard methods during the proliferative stage. More importantly, induced hepatocytes did not express fetal hepatic markers, such as AFP, during the proliferation or differentiation stage on gene or protein levels (Fig. 1D,E). Interestingly, while induced hepatocytes expressed little albumin during proliferation, differentiated cells showed strong levels of mature albumin expression on the gene and protein levels comparable to primary human hepatocytes (Fig. 1D,E).

### Synthetic and transcriptional activity in differentiated hepatocytes

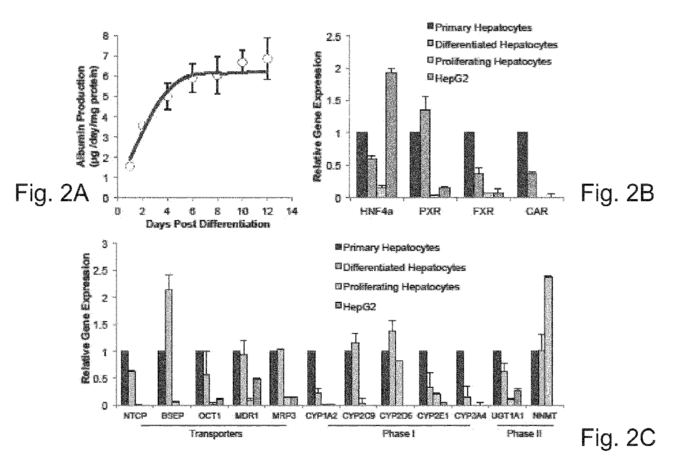

To evaluate the synthetic and transcriptional capacity of the E6 and E7 transduced human hepatocytes, the cells are thawed at passage 12 allowing them to reach confluence and to differentiate in culture. Differentiated hepatocytes produced increasing amounts of albumin, stabilizing after four days of differentiation at 7±1 µg/day/mg protein comparable to 10±5 µg/day/mg protein for cryopreserved hepatocytes (Fig. 2A). All subsequent analysis of differentiated hepatocytes at day four of differentiation are shown, when metabolic function is stabilized. Figure 2B and 2C present quantitative gene expression analysis of nuclear receptors, phase I/II enzymes and drug transporters for freshly isolated primary human hepatocytes, differentiated hepatocytes, proliferating hepatocytes and HepG2 cells. Differentiated hepatocytes gene expression was comparable to freshly isolated primary human hepatocytes. Expression of pregnane X receptor (PXR) and constitutive androstane receptor (CAR), which control the expression of many CYP450 enzymes, was 135% and 36% of that of freshly isolated primary hepatocytes (Fig. 2B). Both these nuclear receptors are seldom expressed in immortalized cell lines. Drug transporters and Phase II enzymes were similarly strongly correlated between differentiated hepatocytes and freshly isolated cells, over an order-of-magnitude higher than proliferating cells or HepG2 (Fig. 2C). Importantly, many of the mature CYP450 enzymes (2D6, 3A4, 2C9, 2E1) showed strong expression in differentiated cells. CYP2D6 was similar to isolated cells for both proliferating and differentiated cells, while CYP2C9 was only expressed in differentiated hepatocytes. CYP3A4 that is responsible for the clearance of over 35% of the drugs on the market was expressed at 15% of freshly isolated cells (Fig. 2C), where most immortalized cells show little to no expression (Hariparsad, N., Carr, B.A., Evers, R. & Chu, X. Comparison of immortalized Fa2N-4 cells and human hepatocytes as in vitro models for cytochrome P450 induction, Drug Metab Dispos 36, 1046-1055 (2008)).

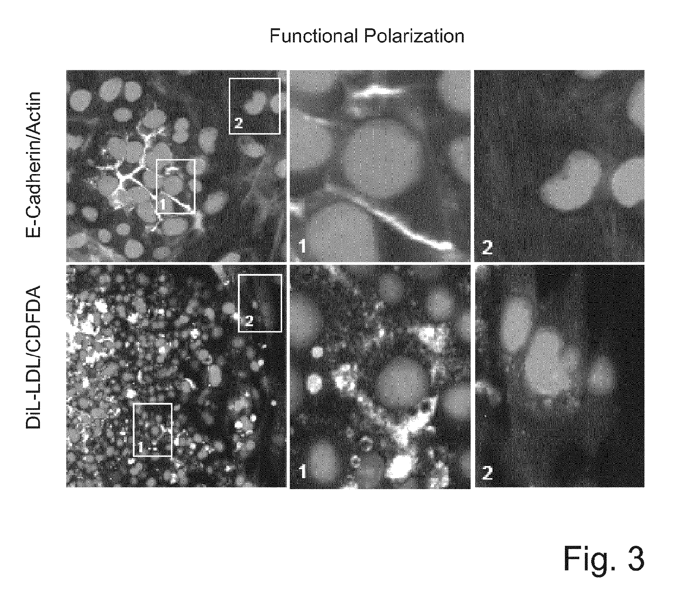

Differentiated hepatocytes show functional polarization Epithelial polarization is critical for the function of mature hepatocytes, which exchange low-density lipoprotein (LDL) and drugs through their basal sinusoidal surface and secrete modified drugs and bile acids into apical bile canaliculi. Polarization is lost during immortalization-induced EMT (S Cannito et al., Antioxidants & redox signaling 2010, 12, 1383-1430). Immunofluorescence staining of differentiated hepatocytes in culture showed distinct colonies of polarized hepatocytes positive for E-cadherin, a lateral surface marker, with actin staining the microvilli-rich sinusoidal surface (Fig.3, A1). Surrounding cells were E-cadherin negative and showed actin stress fibers throughout their cytoplasm (A2). To evaluate the functional activity of these surfaces we exposed differentiated hepatocytes to DiI-LDL and CDFDA. DiI-LDL particles are primarily taken by sinusoidal surface LDL-R 6, while CDFDA is metabolized to fluorescent CDF and secreted to bile canaliculi by MRP2. Functionally polarized hepatocytes were clearly defined by high uptake of DiI-LDL and bright CDF stained bile canaliculi (Fig. 3, B1). In contrast, surrounding cells accumulated CDF in their cytoplasm and showed poor DiI-LDL uptake (B2).

### Drug metabolism and toxicity in induced hepatocytes

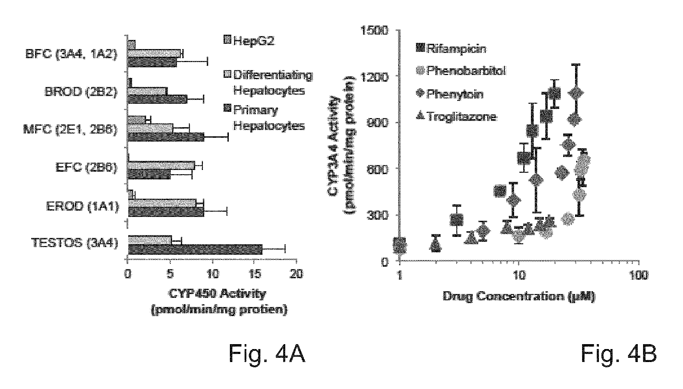

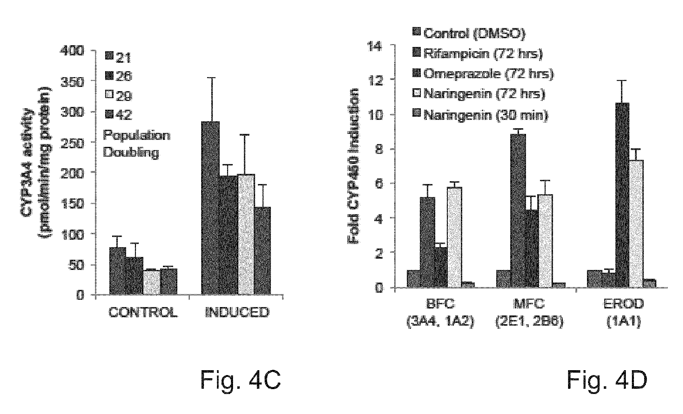

To characterize the metabolic potential of several hepatocytes we compared the CYP450 activity of differentiated hepatocytes to primary human hepatocytes and HepG2 cells (Fig. 4A). We show that CYP450 activity in differentiated hepatocytes is not significantly different from primary cells (p=0.3289) and significantly higher than HepG2 controls (p=0.0007). Metabolism of testosterone, to 6B-hydroxytestosterone by CYP3A4, was 5.2±1 compared to 15.8±3 pmol/min/mg protein in primary human hepatocytes and complete lack of activity in HepG2 cells. Modulation of CYP450 activity by pharmaceuticals or dietary factors underlies drug-drug interactions with serious pharmacological consequences. CYP3A4 activity in differentiated hepatocytes treated with known CYP3A4 inducers showed up to 10-fold dose-dependent induction of testosterone metabolism (Fig. 4B). While CYP450 induction is lost by most immortalization procedures and primary human hepatocytes in culture, CYP3A4 activity and induction in differentiated hepatocytes was stable for over 21 population doublings (Fig. 4C). Other CYP450 induction profile also correlated well with data of literature (Khetani, S.R. & Bhatia, S.N. Microscale culture of human liver cells for drug development, Nature biotechnology 26, 120-126 (2008)). CYP1A1 increased by 10-folds by stimulation with aryl-hydrocarbon receptor (AhR) agonist omeprazole (Fig. 4D). Similarly, pregnane X receptor (PXR) activator rifampicin induced the metabolism of BFC and MFC by 5 and 9-folds, respectably (Fig. 4D). Interestingly, the grapefruit flavonoid naringenin had a time-dependent biphasic effect on CYP450 activity. Naringenin drastically inhibited CYP3A4/5, 2E1, 2B6 and 1A1 after 30 min of incubation. However, after 72 h of incubation with naringenin at the same concentration, we observed a 5 to 7-fold increase in CYP450 activity compared with vehicle-treated cells (Fig. 4D). This is the first demonstration of such behavior by a natural compound.

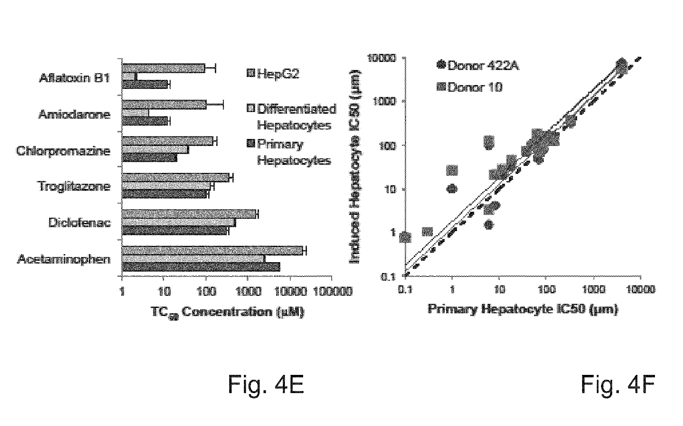

To assess the utility of differentiated hepatocytes for toxicity screening, we compared acute toxicity values to primary human hepatocytes and HepG2 cells (Fig. 4E). Compounds were characterized by TC50, the concentration that caused 50% cell death after 24 hours exposure. Relative hepatotoxicity corresponded well with clinical observations, with aflatoxin B1 showing three orders of magnitude lower TC50 value than acetaminophen (Fig. 4E). Importantly, the TC50 profile was not significantly different from primary cells (p=0.4640) and significantly lower than HepG2 controls (p=0.0012). To assess the cytotoxicity correlation of differentiated hepatocytes to that of primary hepatocytes, the 50% inhibitory concentration (IC50) of 31 compounds was tested in vitro and compared to reported values (Supplement 6A). For the two different donors tested, the IC50 profile correlated well with reported values for primary hepatocytes showing an R2 of 0.99 for two different donors (Fig. 4F).

### Evaluation of toxicological endpoints in differentiated hepatocytes

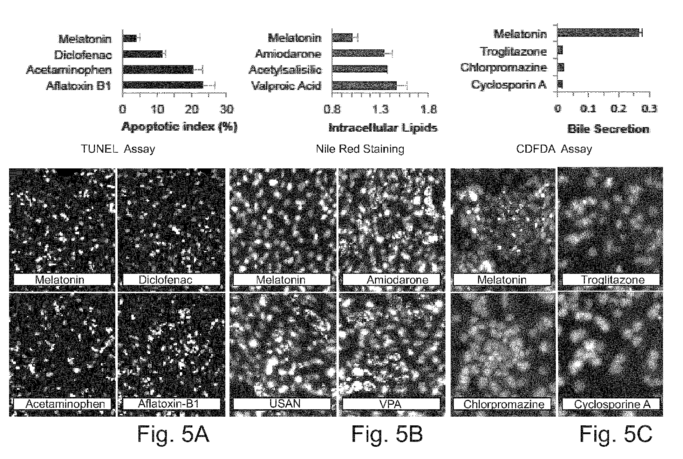

To further characterize the toxicological relevance of induced hepatocytes, we investigated the toxicological endpoint for 12 drugs that are known to cause apoptosis, steatosis and intrahepatic cholestasis in patients. Figure 5A shows the quantification of apoptosis in differentiated hepatocytes exposed for 24 hours to TC20 values of diclofenac, acetaminophen, and aflatoxin B1 compared to melatonin as negative control. Percent apoptosis increased from 4% for melatonin, to 20% positive nuclei for acetaminophen and 23% for aflatoxin B1. Figure 5B shows the quantitation of differentiated hepatocytes exposed to known steatosis-causing agents. After 48 hours of exposure, cultures treated with amiodarone, acetylsalicylic acid (Aspirin), or valproic acid showed a 33% to 47% increase in intracellular lipids compared to control. Finally, figure 5C shows the quantification of bile secretion, as a measure of intrahepatic cholestasis in differentiated hepatocytes exposed to troglitazone, chlorpromazine (Thorazine) and cyclosporine A, compared to melatonin as negative control. All three drugs caused morphological changes leading to loss of bile secretion function (Fig. 5C) and accumulation of fluorescent CDF in the cytoplasm.

### Induced hepatocyte support the full lifecycle of HCVcc

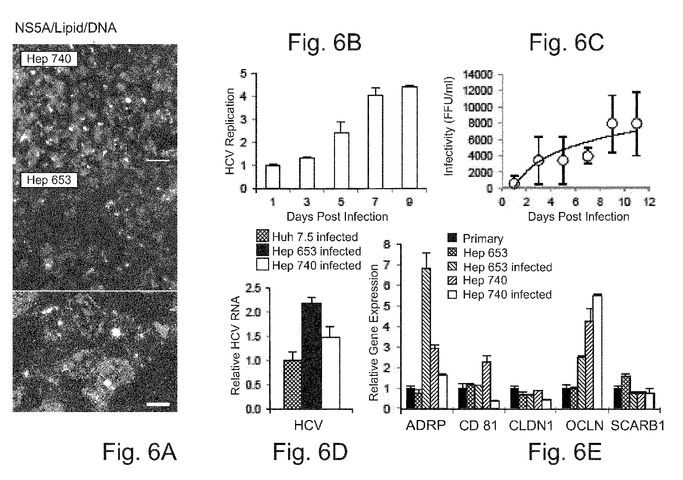

Hepatitis C virus (HCV) infection affects close to 3% of the world's population. The virus infects human hepatocytes in vivo but shows only minimal infectivity of primary human hepatocyte in vitro (A Ploss et al., PNAS 2010, 107, 3141-3145). Up until now, only Huh7 and HepaRG cells showed robust infection of HCV in culture(N Ndongo-Thiam et al. Hepatology 2011, 54, 406-417). To assess whether induced hepatocytes support the full lifecycle of HCVcc we exposed differentiated hepatocytes (donors 740 and 653) to culture medium containing the JC1/RFP variant of HCV (Schaller, T. et al. Analysis of hepatitis C virus superinfection exclusion by using novel fluorochrome gene-tagged viral genomes. J Virol 81, 4591-4603 (2007)). Both donor lines 740 and 653 showed robust NS5A-RFP staining 9 days post-infection (Fig. 6A). Over 80% of the cells were infected in both cultures, compared to 4% to 16% previously reported in literature (A Ploss et al., (supra)). High-resolution confocal microscopy showed that NS5A localized on neutral lipid droplets, an essential step in viral replication (Fig. 6A) (Miyanari, Y. et al. The lipid droplet is an important organelle for hepatitis C virus production. Nat Cell Biol 9, 1089-1097 (2007)). Viral replication increased by 4-folds stabilizing 7 days post-infection (Fig. 6B). Production of infectious viral particles (methods) showed a similar trend, stabilizing after 9 days and reaching 8000 FFU/mL (Fig. 6B). We then carried out quantitative gene expression analysis to compare between induced hepatocyte lines 740, 653 and Huh7.5 cells infected with JC1/RFP. Induced hepatocyte lines 740 and 653 showed 50% and 100% higher levels of HCV RNA compared to Huh7.5 cells (Fig. 6D). Finally, we evaluated the expression of genes critical for HCV entry and replication. Figure 6E shows that induced hepatocyte lines 740 and 653 both support normal levels of expression of HCV entry and replication factors. Interestingly, HCV infection up-regulates the expression of these factors, supporting the continuation of the viral lifecycle.

In the following, the present invention is described in more detail by way of examples. However, these examples are not intended to limit the scope of protection of the present invention in any way.

The examples also refer to several figures, the legends of which are given below:

### Examples:

### Example 1: Inducing the CYP3A4 activity of proliferating hepatocytes

To begin with, hepatocytes that can proliferate are produced by treating primary human hepatocytes with the method described in WO 2009030217A2.

So as to analyze the metabolic capacity of the proliferating hepatocytes described in this invention, the induction of CYP3A4 activity was measured at various doubling figures (PD 23, 32 and 36). For this purpose, the cells were seeded on collagen-coated cell culture vessels in a density of 2.3 x 10⁴ cells/cm² and cultured for 4 days. Thereafter, the cells were treated every day for three days with rifampicin (20 µM), before measuring CYP3A4 activity by way of a luminescence-based P450-Glo assay (Promega). X times the induction (mean value ± standard deviation, n=3) was calculated as the CYP3A4 activity (in RLU / s / well)) of the cells treated with rifampicin divided by the CYP3A4 activity of the control cells. It was possible to induce CYP3A4 activity of the proliferating hepatocytes in all three tested doubling periods. The induction was similar for all doubling times that were analyzed and meets the criteria of the FDA for CYP3A4 induction in human hepatocytes (which is to say greater than four fold).

### Example 2: Isolation and culture

Human primary hepatocytes were genetically modified using Medicyte's proprietary upcyte® technology as described in WO2009/030217A2. Briefly, cells were plated in collagen-coated culture plates at a density of 30,000 cells/cm2 in Hepatocyte Growth Medium (HGM, Medicyte GmbH, Heidelberg, Germany). After 24 h, cells were transduced with viral particles containing the proliferation-inducing genes (A Burkard et al., Xenobiotica 2012, Oct;42(10):939-56) The cells were cultured for an additional 3 to 8 weeks. During this period, the medium was replaced every 2-3 days. Ten days after transduction, proliferating colonies were observed in the transduced cell cultures. To stabilize the epithelial phenotype of the cells, cultures were treated with the MEK1/2 inhibitor U0126 at a concentration range of 2-10µM for a transient period of 1-3 weeks before switching back to HGM medium. Concentration and treatment time varied between different donors and were determined empirically by morphological observation on parallel cultures treated with the different conditions. We observed a high variability in the behavior of the different donors towards this treatment necessitating that the optimal treatment condition had to be determined for each donor. Inhibition of MEK1/2 blocks the ERK1/2 pathway thereby preventing the cells from undergoing the so called epithelial-mesenchymal transition that is induced by the 2D monolayer culture on stiff collagen (Godoy, P. et al. Extracellular matrix modulates sensitivity of hepatocytes to fibroblastoid dedifferentiation and transforming growth factor beta-induced apoptosis, Hepatology 49, 2031-2043 (2009)). This process was evident on cultures that have not been treated with inhibitors where cells adopted a fibroblastoid phenotype. Once, proliferating hepatocytes with epithelial phenotype grew to near confluence they were trypsinized and re-seeded at a density of 20,000 cells/cm². Subsequent passages were performed at a density of 70-80% and with a seeding density of 5,000 cells/cm². The number of PDs was calculated at each passage. For cryopreservation, upcyte® hepatocytes (Medicyte GmbH, Heidelberg, Germany) were resuspended in HGM containing 20% foetal bovine serum and 10% DMSO. The cells were transferred to cryovials and then placed in a Nalgene® "Mr. Frosty" freezing container, which was then placed in a -80°C freezer overnight before storing the cells in liquid nitrogen. The method of freezing and thawing was confirmed to be optimal since the viabilities of all upcyte® Hepatocytes thawed were all higher than 85% and the attachment efficiencies were all above 90% (data not shown).

### Example 3: Quantitative Real Time Polymerase Chain Reaction (qRT-PCR).

RNA was isolated and purified utilizing Macherey-Nagel NucleoSpin RNA II kit according to manufacturer instructions. RNA concentration and purity was determined using NanoDrop ND-1000 spectrophotometer (Thermo Fisher Scientific). Gene expression analysis was carried out utilizing BioRad iScript One-Step RT-PCR Kit on BioRad CFX96 Real-Time System. Gene transcription was evaluated using the ΔΔCt method normalized to Ubiquitin C (UBC), a polyubiquitin precursor, and to RNA from freshly isolated primary hepatocytes from clinical biopsies.

### Example 4: Cytochrome P450 Activity.

To assess CYP450 activity, we measured ethoxyresorufin o-deethylase (EROD), benzyloxyresorufin o-dealkylase (BROD), methoxyresorufin o-demethylase (MROD) and pentoxyresorufin o-dealkylase (PROD) activity as described by Behnia K, Bhatia S, Jastromb N, Balis U, Sullivan S, Yarmush M, Toner M. Xenobiotic metabolism by cultured primary porcine hepatocytes. Tissue Eng. 2000 Oct;6(5):467-79. Briefly, 5 µM of nonfluorescent substrate, specific for a certain CYP enzyme, was added to the culture medium. The rate of appearance of the fluorescent resorufin product secreted into the medium was measured using a fluorescence microplate reader (PerkinElmer Enspire) at 530/590 nm for excitation/emission wavelengths. To further evaluate CYP450 activity, we utilized the method described by Donato MT, Jiménez N, Castell JV, Gómez-Lechón MJ. Fluorescence-based assays for screening nine cytochrome P450 (P450) activities in intact cells expressing individual human P450 enzymes, Drug Metab Dispos. 2004 Jul;32(7):699-706. Briefly, cultures were incubated with various substrates (BFC at 100 µM, CEC at 60 µM, EFC at 30 µM, MFC at 10 µM) for 1 h at 37°c. The reactions were stopped by collection of the incubation medium. Metabolite conjugates formed via Phase II activity were hydrolyzed by incubation of medium samples with ß-glucuronidase/arylsulfatase for 2 hours at 37° C. Samples were diluted 1:1 in quenching solution and the respective fluorescent metabolite formation (HFC for BFC, EFC, MFC; cyano-hydroxycoumarin for CEC; 7-hydroxycoumarin for coumarin) was measured at the appropriate wavelengths. Metabolite formation rate was normalized using total protein amount determined by the Bradford method. For measurement of basal and induced CYP3A4 activities, Cells were seeded at 75,000 cells per well in a 48-well plate. For each measurement cells were seeded in quadruplicates. The cells were allowed to grow for 72 h (without a medium change) and were then either induced with 20 µM rifampicin for CYP3A4 induction or treated with 0.1% DMSO as solvent control. For the analysis of dose induction response, rifampicin was used at the concentrations 0.1, 0.5, 1, 10, 20, and 30 µM. Cells were incubated for 3 days with inducer that was prepared freshly for each day. One day after the induction period, CYP3A4 activity was measured using a 30 min incubation of 250 µM testosterone. Testosterone, 6ß-hydroxytestosterone and the internal standard cortexolone were eluted on a Lichropher RP C18, 5 µm, 4.6 x 250 mm column using a gradient method based on that described by Arlotto MP, Trant JM, Estabrook RW. (1991), Measurement of steroid hydroxylation reactions by high-performance liquid chromatography as indicator of P450 identity and function, Meth Enzymol 206:454-462. The peaks were detected on a UV detector set at 252 nm. Cells were lysed using 0.1 M NaOH and the amount of protein was determined using the Pierce® BCA protein assay kit (Thermo Scientific).

### Example 5: Immunofluorescence Microscopy.

Cultures were fixed with 4% paraformaldehyde for 20 minutes at room temperature after a brief washing with PBS. Cells were permeabilized for 1 hour at room temperature with a 0.1% Triton X-100 blocking buffer containing 2% BSA in PBS, and incubated with primary antibodies for 1 hour at room temperature. Cells were subsequently incubated with secondary antibodies for 1 hour at room temperature following two 45 minute washes. Microscopy was performed utilizing Zeiss LSM 700.

### Example 6: Functional Polarization Assay.

A functional basal surface was ascertained by fluorescence microscopy after a 10-minute incubation of hepatocytes with 10µg/mL DiI-LDL, a fluorescently labeled low density lipoprotein. Functional bile canaliculi was detected after a 10-minute incubation with 2µg/mL 5(6)-carboxy-2',7' -dichlorofluorescein diacetate (CD-FDA). Upon diffusion into hepatocytes, CDFDA is cleaved by cytoplasmic esterases, forming a fluorescent carboxydichlorofluorescein (CDF) molecule. CDF is then transported into bile canaliculi by MRP2 in polarized hepatocytes.

### Example 7: CYP3A4 Activity as Function of Drug Concentration.

Cells were seeded at 75,000 cells per well in in collagen Type I-coated 48-well plates. For each measurement cells were seeded in quadruplicates. The cells were cultured for 3 days prior to medium change. After this time, the cells were treated daily for 3 days with a range of concentrations of test compound or the solvent control (0.1% PBS for phenobarbital controls and 0.1% DMSO for all other compounds). Cells were induced with 20 µM rifampicin (for CYP3A4 induction), 2 mM phenobarbitone (for CYP2B6 induction), or 50 µM omeprazole (for CYP1A2 induction). The inducers were dissolved in DMSO and the final concentration was 0.1% - concurrent control incubations included 0.1% DMSO. For determination of the RIS for CYP3A4, cells were treated daily for 72 h with 1, 5, 10, 20, 25, 30 µM carbamazepine, 0.1, 1, 1.5, 5, 10, and 20 µM nifedipine, 100, 250, 500, 1000, 1500, and 2000 µM phenobarbital, 1, 10, 50, 100, 150, and 200 pioglitazone, 0.1, 0.5, 1, 10, 20, and 30 µM rifampicin, 5, 10, 15, 20, 50, and 100 µM troglitazone, 1, 10, 100, 1000, 1500, and 2000 µM dexamethasone. At the end of the induction period, the cells were washed with PBS and 0.2ml 250µM testosterone in KHB was added to each well and incubated for 30min. The supernatant was transferred to a fresh 96-well plate and processed for HPLC analysis. The remaining cell cultures were again washed with PBS and then incubated with 500µM bupropion in KHB for 1h. The supernatant was transferred to a fresh 96-well plate and processed for HPLC analysis. The viability of the cultures was then measured using the MTS assay (carried out according to the supplier's protocol (Promega, Mannheim, Germany)). Briefly, a volume of 0.2ml MTS was added per well and incubated for 1h in a humidified incubator at 37°C, under an atmosphere of 5% CO2/95% air. The absorbance was read at 490nm. Proteins were dissolved in 0.2ml lysis buffer (8.76 mg/ml NaCl; 0.2mg MgCl₂.6H₂O; 1% (v/v) NP40; 50mM Tris-HCl) and the protein content was measured using the Pierce assay.

Example 8: Toxicity Dose Response Assay. Cultures were incubated with compounds of different concentrations dissolved in culture medium for a period of 24 hours. Cell viability was subsequently determined utilizing LIVE/DEAD Viability/Cytotoxicity Kit according to manufacturer instructions (Molecular Probes). In short, cultures were incubated for 25 minutes at 37°C with 2µM Calcein AM and 3µM Ethidium homodimer-1 in PBS. Fluorescence microscopy was performed utilizing Zeiss LSM 700.

### Example 9: Toxicity Mode of Action Assays.

Quantitation of apoptotic cells within the culture was performed utilizing DeadEnd Fluorometric TUNEL System (Promega). The DeadEnd system catalytically incorporates fluorescein- 12-dUTP at 3' -OH DNA ends utilizing the enzyme Terminal Deoxynucleotidyl Transferase (TdT). Cultures were treated with either 1 mM acetaminophen, 300 µM diclofenac, or 5 mM melatonin dissolved in culture medium for 24 hours. Subsequently, cultures were fixed with 4% formaldehyde in PBS for 24 minutes at 4°C. After two brief washes with PBS, cells were permeabilized for 5 minutes with 0.2% Triton X-100 in PBS for 5 minutes. After additional washes, cultures were equilibrated at room temperature for 10 minutes with Equilibration Buffer and then treated with TdT reaction mixture for 60 minutes at 37°C. The reaction was stopped utilizing 2X SSC for 15 minutes, cells washed with PBS, and counterstained with DAPI. Fluorescence microscopy was performed utilizing Zeiss LSM 700. Measurement of intracellular lipids in steatotic cells was performed utilizing Nile Red staining. Cultures were incubated with either 5 µM amiodarone or 5 mM melatonin for 24 hours in culture medium. Cells were subsequently treated with 1 µM Nile Red in HBSS for 20 minutes, and washed with PBS. Fluorescence microscopy was performed utilizing Zeiss LSM 700.

### Example 10: Cytochrome P450 Induction and Inhibition.

Cultures were incubated with 50 µM omeprazole, 25 µM rifampicin, or 200 µM naringenin dissolved in culture medium for 4 days prior to performance of Cytochrome P450 assay. All data were normalized to vehicle-only controls.

### Example 11: HCV Replication and Infectivity.

Media was collected from JC1/RFP HCV infected Huh7.5 after 72 hours of incubation and filtered. Differentiated hepatocyte cultures were incubated with differentiation media containing 10% filtered HCV supernatant for 24 hours. Media was subsequently changed daily to untreated differentiation media. To determine HCV replication, fluorescence microscopy imaging for NS5A-RFP was performed utilizing Zeiss LSM 700. For infection studies, focus-forming units were imaged and counted. For colocalization studies, lipids were stained utilizing LipidTOX green neutral lipid stain (Invitrogen H34158). Media was removed from cultures and subsequently washed briefly with PBS, then fixed as mentioned above. LipidTOX Green neutral lipid 1000x stain was diluted 1:1000 in PBS. Cells were incubated for 30 minutes at room temperature and imaged with high-resolution confocal microscopy.

Fig. 1. Induction of reversible proliferation in primary human hepatocytes.

(A) Phase microscopy identifies colonies of proliferating hepatocytes following viral transduction (*solid boundary*)*.* (B) Gene expression analysis of HPV-transformed HeLa cells, induced hepatocytes, and primary human hepatocytes. Induced hepatocytes express 1×10⁶-fold less HPV E6/E7 than HeLa cells. Transduction also up-regulates expression of OSM receptor, IL6ST. (C) Proliferation curve of induced proliferating hepatocytes post cryopreservation, as well as differentiated hepatocytes allowed to reach confluence. Cell doubling time was 39 hours. (D) Gene expression analysis of freshly isolated primary human hepatocytes, and HepG2 liver cancer cell line, compared to induced hepatocytes during proliferation and differentiation stages. Induced hepatocytes do not express the fetal liver marker alpha-fetoprotein (AFP) like liver tumor cells, but gain albumin expression post-differentiation. (E) Phase micrographs of induced hepatocytes during proliferation and differentiated stages with corresponding immunofluorescence micrographs of AFP and albumin, compared to primary hepatocytes and HepG2.

Fig. 2. Induced hepatocytes show high levels of gene expression comparable to primary cells.

(A) Rate of albumin synthesis over two weeks in culture. Secretion stabilizes four days post differentiation (B, C) Gene expression analysis of freshly isolated primary human hepatocytes, and HepG2 liver cancer cell line, compared to induced hepatocytes during proliferation and four days post-differentiation. HNF4α, hepatocyte nuclear factor 4 alpha; PXR, pregnane X receptor; FXR, farnesoid X receptor; CAR, constitutive androstane receptor; NTCP, sodium-dependent bile acid transporter; BSEP, bile salt export pump; OCT, organic cation transporter; MDR1/P-gp, multidrug resistance protein 1; MRP3, multidrug resistance protein 3; UGT, UDP glycosyltransferase; NNMT, nicotinamide N-methyltransferase.

Fig. 3. Differentiated hepatocytes show functional polarization.

(A) Immunofluorescence micrograph of E-Cadherin, a lateral surface marker, in differentiated hepatocytes counter-stained for actin, which primarily stains microvilli on the basal surface in polarized cells. Cultures show distinct polarized cell nodules (1) amid non-polarized E-cadherin negative cells (2). (B) Functional polarization in differentiated hepatocytes. CDFDA staining shows the accumulation of green CDF in functional bile canaliculi on the apical surface of polarized cells (1). DiI-LDL particles are taken primarily by the LDL-R expressed on the basal surface of polarized cells. The assays shows basal-apical polarized cell nodules (1) surrounded by non-polarized cells (2). LDL, low-density lipoprotein.

Fig.4. Differentiated hepatocytes show high levels of CYP450 activity and drug metabolism in vitro

(A) CYP450 activity of differentiated hepatocytes compared with HepG2 cells and primary human hepatocytes. CYP450 activity of differentiated hepatocytes is comparable to primary human cells. (B) CYP3A4 activity in induced differentiated hepatocytes at different dosages. (C) Basal and induced CYP3A4 activity in differentiated hepatocytes at various population doublings showing stability of function. (D) Induction of CYP450 activity in differentiated hepatocytes via different compounds. Rifampicin is a CYP3A4 and 2E1 agonist, omeprazole is a strong inducer of CYP1A1/2⁵⁶. Naringenin shows a time-dependent biphasic effect on CYP3A4 and 2E1 activity, inhibiting activity in minutes but inducing activity following 72 hours of exposure. (E) TC₅₀ values of different pharmaceutical agents obtained from 24-hour dose-response studies in differentiated hepatocytes, HepG2 cells and primary human hepatocytes. TC₅₀ values of differentiated hepatocytes are closely match primary human hepatocyte values. (F) Comparison of the IC₅₀ values of 31 compounds between cultures of differentiated hepatocytes and primary hepatocytes. IC₅₀ values of differentiated hepatocytes correlate well with previously reported values for primary hepatocytes.

Fig. 5. Differentiated hepatocytes show critical toxicological end-points in vitro.

(A) Apoptosis of differentiated hepatocytes following 24 hours of exposure to apoptosis-causing drugs or melatonin (negative control). Apoptosis was quantified using TUNEL and is shown as percent positive nuclei. (B) Lipid accumulation (steatosis) of differentiated hepatocytes following 24 hours of exposure to steatosis-causing drugs or melatonin (negative control). Apoptosis was quantified using Nile Red staining and shown relative to control. (C) Intrahepatic accumulation of bile acids (cholestasis) of differentiated hepatocytes following 24 hours of exposure to cholestasis-causing drugs or melatonin (negative control). Cholestasis was quantified using CDFDA staining as number of functional bile canaliculi.

Fig. 6. Multiple lines of induced hepatocytes support robust HCVcc infection.

(A) Fluorescence micrographs showing JC1/RFP replication in two different genotypes of induced hepatocytes (lines 743 and 653) following exposure to supernatant containing the virus. JC1/RFP contains a NS5A viral protein fused to RFP (Zhong, J. et al. Robust hepatitis C virus infection in vitro. Proceedings of the National Academy of Sciences of the United States of America 102, 9294-9299 (2005)). Cells were counterstained for neutral intracellular lipids using LipidTOX Bar = 50 µm. High-resolution confocal micrograph (*bottom*) shows co-localization of NS5A and lipid droplets, a hallmark of viral infection. Bar = 10 µm. (B) HCV replication in differentiated hepatocytes quantified by NS5A fluorescence over nine days. (C) Extracellular infectivity of the secreted virus in differentiated hepatocytes measured by Focus Forming Unit (FFU) assay (D) Quantitative comparison of HCV RNA expression at day 9 in two lines of induced hepatocytes compared with Huh7.5 hepatoma cells electroporated with viral RNA. (E) Quantitative gene expression analysis of HCV entry receptors in primary human hepatocytes compared to two genotypes of induced hepatocytes, at day 9. Both lines show comparable expression of entry receptors, which are significantly induced during HCV infection.

## Claims

**1.** Use of proliferating hepatitis infected hepatocytes for carrying out hepatitis test procedures, **characterized in that** these proliferating hepatocytes are derived from primary cells of humans or other mammals comprising
a) a proliferation gene, in particular a cellular and/or a viral proliferation gene; and/or
b.) at least one cellular factor is inactivated that induces cell division arrest,
and/or
c.) are transiently immortalized.

**2.** Use of proliferating hepatitis infected hepatocytes according to claim 1, wherein the hepatitis is caused by virus, fungus, parasite or bacteria.

**3.** Use of proliferating hepatitis infected hepatocytes according to claim 1 or 2, **characterized in that** these proliferating hepatocytes comprise at least four Phase I enzymes selected from the group consisting of CYP1A2, CYP2C9, CYP2C19, CYP2D6, CYP2E1, CYP3A4, CYP1A2, CYP2A6, CYP2B6, CYP2C8, CYP1A1, CYP3A5, CYP3A7 and CYP4A11.

**4.** Use of proliferating hepatitis infected hepatocytes according to claim 1, **characterized in that** this cellular proliferation gene is selected from the group consisting of myc, jun, ras, src, fyg, myb, E2F and Mdm2 and TERT, or the viral proliferation gene is selected from the group E6 and E7 of papillomaviruses such as HPV; the large and small TAg of polyomaviruses such as SV40, JK virus and BC virus; the E1A and E1B proteins of adenoviruses, EBNA proteins of the Epstein Barr virus (EBV),;as well as HTLV and Herpesvirus saimiri.

**5.** Use of proliferating hepatocytes according to claim 1, **characterized in that** the viral proliferation genes are E6 and E7 of HPV or BPV, in particular HPV16 and HPV18 and HPV 31, 33, 35, 39, 45, 51, 52, 56, 58, 59, 68, 73 and 82 and/or HPV6 and HPV11 as well as HPV 40, 42, 43, 44, 54, 61, 70, 72 and 81.

**7.** Use of proliferating hepatitis infected hepatocytes according to claim 1, **characterized in that** the cellular factor is selected from the group consisting of p53, p16, pRb, p107, p130 or the respective upstream or downstream factors thereof, or proteins binding thereto in the pathway, and the inactivation of such cellular factors takes place by way of the expression of dominant negative mutants or by the inhibition of gene expression of these factors using antisense oligonucleotides, RNAi molecules, morpholinos, ribozymes, or by way of gene knockout, by the action of specific antibodies or by chemical inhibitors.

**8.** Use of proliferating hepatitis infected hepatocytes according to claim 1, **characterized in that** the transient immortalization takes place by way of i.) a polypeptide having cell immortalization activity, ii.) a polypeptide that synthesizes telomeric DNA at chromosomal ends, or a respective fusion peptide thereof.

**9.** Use of proliferating hepatitis infected hepatocytes according to claim 1, **characterized in that** the transient immortalization takes place by way of i.) a polypeptide having cell immortalization activity selected from the group of an expression product according to claim 7 or 8.

**10.** Use of proliferating hepatitis infected hepatocytes according to claim 5, **characterized in that** the transient immortalization takes place by way of ii.) a polypeptide that synthesizes telomeric DNA at chromosomal ends which is selected from the group consisting of telomerase, telomerase reverse transcriptase (hTERT), P140, P105, p48 and p43.

**11.** Use of proliferating hepatocytes according to claim 1, **characterized in that** the transient immortalization takes place by way of a fusion peptide, wherein the first part is a transport polypeptide, in particular VP22, HIV TAT, (HIV) REV, Antennapedia polypeptide, Penetratin, Engrailed, Hoxa-5, a polymer made of L-arginine or D-arginine amino acid residues, a polymer made of L-lysine or D-lysine amino acid residues, transcription factors such as BETA2/neuro D, PDX-1, nuclear localization signal, histone-derived peptides, a polymer made of cationic macromolecules, FGF-1 and FGF-2, lactoferrin, and the second part is a polypeptide according to either claim 6 or 7.

**12.** A method for producing an assay or test system, comprising the following steps:
a.) providing a substrate material;
b.) immobilizing or fixing or suspending proliferating hepatitis infected hepatocytes on this substrate material;
c.) infecting the hepatocytes from b.) with bodily fluid from a patient suffering from hepatitis and obtaining hepatitis infected hepatocytes and culturing or enriching;
c.') optionally portioning obtained hepatitis infected hepatocytes resulting in one or more variant of the hepatitis infected hepatocytes;
bringing at least one cell from c.) or c') in contact with an agent.

**13.** A method for producing a cell bank, comprising the following steps:
a.) providing a substrate material;
b.) immobilizing or fixing or suspending proliferating hepatocytes on this substrate material;
c.) infecting the hepatocytes from b.) with bodily fluid from a patient suffering from hepatitis and obtaining hepatitis infected hepatocytes and culturing or enriching;
c.') optionally portioning obtained hepatitis infected hepatocytes resulting in one or more variant of the hepatitis infected hepatocytes.

**14.** A method for characterization of an external cell, comprising the following steps:
a.) providing a substrate material;
b.) immobilizing or fixing or suspending proliferating hepatitis infected hepatocytes on this substrate material,
c.) obtaining several variants of proliferating hepatitis infected hepatocytes through culturing and optionally portioning,
d.) optionally, bringing at least one cell from c.) in contact with an agent or
e.) optionally, comparing at least one cell from c.) with an external hepatocyte in order to identify the variant of the hepatitis infected hepatocytes.

**15.** A method for producing an assay according to claim 12 or 13, **characterized in that** the agent is selected from the group of chemical and biological active substances and drugs.

**16.** Use of an assay or cell bank comprising proliferating hepatitis infected hepatocytes for identifying, stratifying of patients or individuals, in particular in responder, non-responder or relapser.

**17.** Use of an assay or cell bank comprising proliferating hepatitis infected hepatocytes for risk stratification of hepatitis for the execution of clinical decisions, particularly advanced treatments and therapies using drugs, particularly in intensive care or emergency care, to include the decision to hospitalize the patient and/or for the risk stratification of hepatitis for the prognosis, early detection and detection by differential diagnosis, assessment of the severity, and assessment of the course of the disease concomitant with the therapy.
